# EUROPEAN PATENT APPLICATION

(11) **EP 2 158 899 A1**
(43) Date of publication of application: **03.03.2010**
(21) Application number: 09167641.1
(22) Date of filing: 11.08.2009
(51) Int. Cl.: A61K 9/00, A61K 9/70, A61K 47/32

(54) **Improved composition for the topical transmission of active ingredients into the human or animal body**

(30) Priority: 29.08.2008 IT MI20081552
(71) Applicant: BIOFARMITALIA S.p.A., 20144 Milano (IT)
(72) Inventor: Di Schiena, Michele Giuseppe, 20087, ROBECCO SUL NAVIGLIO (MI) (IT); Lombardo, Paola, 24060, COSTA MEZZATE (BG) (IT)
(74) Representative: Frignoli, Luigi

(57) **Abstract**

Improved composition for transmission into the human or animal body of pharmaceutical, cosmetic or nutritional active ingredients, said transmission occurring by topical application on the skin and/or mucous membrane of the body.

## Description

The present invention concerns a composition in the form of an emulsion (solid or fluid), gel, lotion or active component of a patch, applicable on the skin or mucous membrane of the human or animal body, suitable for permitting and maintaining high transmission into the body of the active ingredients (pharmaceutical, cosmetic or nutritional) present in the composition.

Compositions of said type are already well known: they are in the form of creams, fluid emulsions, lotions, gels or active components distributed on the surface of a patch, and are applied on the skin or mucous membrane of the human or animal body to transmit the active ingredients into the body gradually and in a constant prolonged manner.

The known compositions have the drawback that said transmission occurs in a slow and partial manner, thus not guaranteeing complete and rapid use of the active ingredient, therefore reducing the effectiveness of the compositions and increasing the cost of the treatment.

The patent EP 0880965 A describes a topical formulation comprising nimesulide and a sodium acrylate copolymer. Since the nimesulide has the serious problem of giving the skin treated with it an intense yellow colouration, the sodium acrylate copolymer present in the formulation of said patent has the sole purpose of preventing or significantly reducing the problem of the colouration. Said European patent does not describe the use of any other pharmaceutical ingredient in addition to nimesulide (with regard to which only the problem of intense colouration is taken into consideration).

The patents US2006/0079640 and US5344655 describe pharmaceutical formulations comprising *both pharmaceutical, cosmetic or nutrient substances and also sodium acrylate copolymer* having the purpose of permitting *an enhanced percutaneous absorbability of the pharmaceutical components of such formulations.* However, the experiments performed by the present applicants have shown that *the percutaneous absorption of* a *medicament comprising the most commonly used sodium copolymers, including all those mentioned in the above referred US patent publications, show a slow percutaneous absorption of the active ingredients, so that a substantial amount of the active ingredients can be absorbed only* if said pharmaceutical formulations are kept in contact with the skin for very long periods of time.

One object of the invention is therefore to produce a composition of the type mentioned which ensures a rapid and substantial topical transmission of the active ingredient contained in it, thus resulting more effective than the corresponding known topical compositions and reducing the cost of treatment of the body with the active ingredients.

Said composition comprises at least one pharmaceutical, cosmetic or nutritional active ingredient, and a sodium acrylate copolymer (in the form of a fluid emulsion, a cream, a gel, a lotion or an active component on a surface of a patch), said sodiuum acrylate polymer being a sodium salt of a polymer of acrylic acid, methacrylic acid or one of their simple esters such as specified herebelow.

Preferably said copolymer is present in a quantity of between 0.005% and 99.9%, the percentages being w/w of the active ingredient.

In this invention, the term "sodium acrylate copolymer" indicates the *per se* known (see: International Cosmetic Ingredient Dictionary and Handbook, 10th Ed., 2004, Vol.2, page 1696) sodium salts of a polymer consisting of acrylic acid, methacrylic acid or one of their simple esters, for example the one produced by CIBA SPECIALTY CHEMICALS and sold under the registered name SALCARE SC91 and SALCARE AST.

A peculiar characteristic of these particular copolymers is their high capacity to emulsify oil-in-water systems and water-in-oil systems, thus permitting a wide range of topical compositions.

A further peculiar characteristic of these particular copolymers is that they perform their emulsifying activity at ambient temperature, hence there are no additional costs for heating; this peculiar characteristic is very useful for obtaining topical compositions with thermolabile active ingredients as is the case, for example, with many vegetable extracts, essential oils, animal extracts, vitamins, hormones, enzymes, ferments, antibiotics, antivirals, etc.

The composition according to the invention can advantageously comprise other excipients on condition that they are physiologically acceptable, compatible with the envisaged use and compatible with the sodium acrylate copolymer.

Preferred excipients are chosen from the group consisting of water, alcohols, natural or synthetic oils, gelatinizing products, suspending agents, emulsifying agents, thickening agents, inert powders, natural and synthetic polymers, sweeteners, aromas, fragrances, colouring agents, preservatives, compounds favouring epithelial and connective absorption.

In order to clarify the characteristics of the compositions according to the present invention, some non-limiting examples thereof will now be described in which the sodium acrylate copolymer used as "polymeric excipient" is of the type commercially available under the brand name SALCARE SC91 and having the following technical specifications:
INCI NAME: Sodium Acrylate Copolymer, mineral oil, PPG-1 trideceth-6;
Appearance at 20°C: liquid dispersion;
pH: 6.8-8.0;
Brookfield viscosity (solution 2% in water): 20000-40000 cPs;
Mean Sodium Acrylate Copolymer content: 50%;
Acute toxicity: LD50 (oral in rats) > 2000 mg/Kg.

### EXAMPLE 1

### Preparation of a caffeine-based patch for the treatment of cellulite

58 Kg of non cross-linked solvent acrylic adhesive (for example Duro-tak 387-2353 adhesive by National Starch & Chemical), 500 g of "polymeric excipient" SALCARE SC91 and 2.9 Kg of anhydrous caffeine diluted in H₂O in proportion 1/1 (obtained by stirring at 80°C for 5 minutes in an appropriate stirrer) are placed cold in a container and mixed for 10 minutes at low speed (120 r.p.m.) with a propeller stirrer until a uniform mass is obtained. It is left to rest to allow for the release of any air bubbles. Using a patch coating machine and with the aid of a compressed air pump, the mixture is placed in the doctor blade with rotating cylinder after setting the thickness of the blade to approximately 300 microns. The mixture is filmed on silicone-coated polyester which passes through four oven stations, the first one set to 40°C, the second to 50°C, the third to 70°C and the fourth to 80°C, at a speed of 8 metres per minute. At the oven outlet the film is completely solvent-free as the solvents have evaporated in the oven stations, the adhesive mass is transparent and slightly opalescent, highlighting the presence of polymeric excipient, and the weight of the adhesive mass is approximately 60 g/m². The polyester on which the adhesive film has been coated and dried is coupled to a 65 g/m² polythene film and wound on a reel. The result is that the adhesive film clings to the polythene forming an adhesive film containing caffeine (approximately 0.1 mg/cm²) and polymeric excipient (approximately 15 mcg/cm²) protected by the silicone-coated polyester. The reel obtained is then punched into 3 cm diameter round patches and pouched. Said patches were tested to verify the caffeine release curve over time and the result obtained showed that the sodium acrylate copolymer had a markedly positive influence on the process of release and penetration of the active ingredient through the skin. More specifically it was found that with use of the patch described above, transmission (into the body via the skin) of 0.72 mcg/cm²/hour is obtained. With the use of analogous patches of known type (without the sodium acrylate copolymer, of the type mentioned above) a transmission of 0.59 mg/cm²/hour is obtained, hence the difference is substantial.

### EXAMPLE 2

### Preparation of an ichthyol-based patch for the treatment of anal fistulae

8.7 Kg of non cross-linked solvent acrylic adhesive (for example Duro-tak 387-2353 adhesive by National Starch & Chemical) and 323 g of pure ichthyol are placed cold in a container and mixed with a propeller stirrer until a uniform mass is obtained. 80 g of "polymeric excipient" SALCARE SC91 are introduced still under stirring. It is left to rest to allow for the release of any air bubbles. Using a patch coating machine and with the aid of a compressed air pump, the mixture is placed in the doctor blade with rotating cylinder after setting the thickness of the blade to approximately 300 microns. The mixture is filmed on a film of silicone-coated polyester which passes through four oven stations, the first one set to 40°C, the second to 50°C, the third to 70°C and the fourth to 80°C, at a speed of 8 metres per minute. At the oven outlet the film is completely solvent-free as the solvents have evaporated in the oven stations, the adhesive mass is blackish, highlighting the presence of pure ichthyol at 10%, and the weight of the adhesive mass is approximately 90 g/m². The polyester film on which the adhesive film has been coated and dried is coupled with a 100 g/m² polyester and viscose non-woven fabric and wound on a reel. The result is that the adhesive film clings to the non-woven fabric forming an adhesive fabric containing ichthyol at 10% protected by the silicone-coated polyester. Subsequently the reel obtained is punched into 4 cm diameter round patches and pouched. Said patches were tested to verify the quantity of pure ichthyol present by means of HPLC and each patch was found to contain 11.3 mg of ichthyol equal to 10% of the total adhesive mass.

The tests performed subsequently by organised clinical facilities on persons with anorectal fistulae or abscesses showed a marked effectiveness of the product: reabsorption of the abscess with complete resolution of the pain and tissue symptomatology. Over a period of 8-10 hours, there is a progressive reduction in the characteristic symptoms: anal-perianal pain and swelling, irritation and reddening of the perianal skin (itchiness, smarting and pus).

### EXAMPLE 3

### Preparation of a diclofenac diethylammonium-based patch for the treatment of articular pain

27.5 kg of an elasticising agent (for example tributyl citrate TBC) and 3.5 kg of diclofenac diethylammonium are placed cold in a propeller mixer and dispersed, stirring for 10 minutes at ambient temperature and at approximately 80 r.p.m.; while still mixing, 68.9 kg of water-based acrylic adhesive (for example EUDRAGIT NE 40D adhesive) and 0.586 g of "polymeric excipient" SALCARE SC91 are added, continuing to mix until a uniform mass is obtained. It is left to rest to allow for the release of any air bubbles. Using a patch coating machine and with the aid of a compressed air pump, the mixture is placed in the doctor blade with rotating cylinder after setting the thickness of the blade to approximately 300 microns. The mixture is filmed on a film of silicone-coated polyester which passes through four oven stations, the first one set to 40°C, the second to 50°C, the third to 70°C and the fourth to 100°C, at a speed of 6 metres per minute. At the oven outlet the film is completely solvent-free as the solvents have evaporated in the oven stations and the thickness of the adhesive mass is approximately 190 microns (200 grams per square metre). The polyester film on which the adhesive film has been coated and dried is coupled with a 70 g/m² polyester and viscose non-woven fabric and wound on a reel. The result is that the adhesive film clings to the non-woven fabric forming an adhesive fabric containing diclofenac diethylammonium in a concentration of 1.2 mg/cm². Subsequently the reel obtained is punched into 10x14 cm rectangular patches and pouched.

### EXAMPLE 4

### Preparation of an ibuprofen lysine-based patch for the treatment of articular and muscular pain

28 kg of demineralised water, 0.120 kg of parabenes (preservatives), 4.4 kg of polyvinyl alcohol and 0.5 kg of carboxymethyl cellulose are placed in a mixer heated to 70°C and mixed until a uniform mass is obtained which is cooled to ambient temperature and then poured into a blender in which a mass formed of 900 g of ibuprofen lysine, 18 kg of sodium alginate and 500 g of "polymeric excipient" SALCARE SC91 is added cold (over a period of approximately 15-20 minutes). It is stirred for 15-20 minutes in both directions.

The gel is spread by a doctor blade with twin rotating cylinders on a strip of non-woven fabric formed of viscose fibres (50%) and polypropylene fibres (50%), 150 g/m², with a thickness of 200 microns and a density of 87 g/dm³.

The protective backing 4 consists of a sheet of silicone-coated polyester 75 g/m².

The composite strip fed out of the rollers is conveyed to a machine where it is cut and punched, for example, assuming the form of a 10x15 cm rectangular patch.

The patch thus obtained is particularly effective for applications on badly irritated areas, as the refreshing action of the gel offers an immediate soothing and calming effect, while the ibuprofen lysine has an anti-inflammatory and pain-relieving action.

### EXAMPLE 5

### Preparation of a vitamin E-based cream for the treatment of vitamin E deficiency

79% of purified water according to the Official Pharmacopoeia, 15% of squalane (eudermic oil) and 3% vitamin E (natural) are mixed.

Stirring vigorously, the 3% of "polymeric excipient" SALCARE SC91 is added, the percentages indicated being w/w of the cream.

After approximately 5 minutes the stirring speed is reduced and stirring is continued at average speed until complete formation of the cream.

Stirring is stopped and after 12 hours' rest the cream is mixed slowly for 30 minutes.

### EXAMPLE 6

### Vitamin-E based fluid emulsion for the treatment of vitamin E deficiency

The same procedure as in example 5 is followed reducing the squalane to 5% and the "polymeric excipient" SALCARE SC91 to 1%; the water is consequently increased.

## Claims

1. Improved composition for the topical transmission of active ingredients into the human or animal body, comprising at least one pharmaceutical, cosmetic or nutritional active ingredient and at least one sodium acrylate copolymer, **characterised in that** said sodium acrylate copolymer is a sodium salt of a polymer of acrylic acid, methacrylic acid or one of their simple esters, as defined in the International Cosmetic Ingredient Dictionary and Handbook.

2. Composition as claimed in claim 1, **characterised in that** said copolymer is present in a quantity of between 0.005% and 99.9%, the percentages being w/w of the active ingredient.

3. Composition as claimed in claims 1 or 2, which is in the form of a fluid emulsion, a cream, a gel, a lotion or an active component distributed on the surface of a patch.
